(19) Europäisches Patentamt
European Patent Office
Office européen des brevets

(11) Veröffentlichungsnummer: **0 019 872**
**B1**

(12) # EUROPÄISCHE PATENTSCHRIFT

(45) Veröffentlichungstag der Patentschrift: **16.06.82**

(51) Int. Cl.³: **C 09 B 57/14**, C 07 D 311/78, C 07 D 335/04

(21) Anmeldenummer: **80102880.4**

(22) Anmeldetag: **23.05.80**

(54) **Verfahren zur Herstellung von Benzoxanthen- und Benzothioxanthenfarbstoffen.**

(30) Priorität: **01.06.79 DE 2922374**

(43) Veröffentlichungstag der Anmeldung: **10.12.80 Patentblatt 80/25**

(45) Bekanntmachung des Hinweises auf die Patenterteilung: **16.06.82 Patentblatt 82/24**

(84) Benannte Vertragsstaaten: **CH DE FR GB LI**

(56) Entgegenhaltungen:
**DE-B-2 025 291**
**US-A-3 772 333**

(73) Patentinhaber: **HOECHST AKTIENGESELLSCHAFT, Postfach 80 03 20, D-6230 Frankfurt/Main 80 (DE)**

(72) Erfinder: **Elinkmann, Hans-Gerd, Kallestrasse 6, D-6000 Frankfurt am Main (DE)**
Erfinder: **Paszthory, Emmerich, Dr., Königsteiner Strasse 18, D-6238 Hofheim am Taunus (DE)**

EP 0 019 872 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Benzoxanthen- und Benzothioxanthenfarbstoffen

Aus der DE-A 20 25 291 ist es bekannt, Isomerengemische der Benzoxanthen- oder Benzothioxanthenfarbstoffe der allgemeinen Formeln

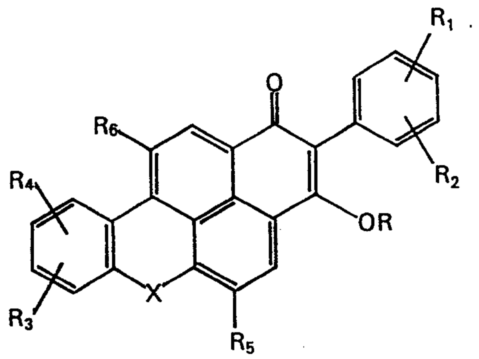

Ia

und

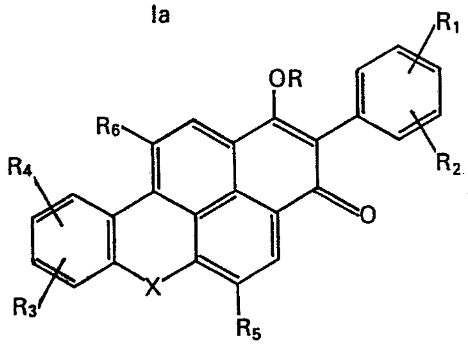

Ib

worin X ein Sauerstoff- oder Schwefelatom, R einen Benzolsulfonylrest, $R_1$ und $R_2$ Wasserstoff- oder Halogenatome, Alkylphenyl-, Acyl-, Acyloxy-, Carbalkoxy-, Alkoxy-, Nitro-, Alkylthio-, oder Alkylsulfonylgruppen oder zusammen einen annellierten Benzolring, $R_3$ und $R_4$ Wasserstoff- oder Halogenatome, Phenylgruppen, Alkyl-, Alkoxy-, Carbalkoxygruppen mit 1 bis 4 C-Atomen oder Nitrilgruppen, $R_5$ und $R_6$ Wasserstoffatome oder gegebenenfalls substituierte Alkoxygruppen mit 1 bis 4 C-Atomen bedeuten, herzustellen, indem man Benzoxanthen- oder Benzothioxanthen-3,4-dicarbonsäureanhydride der allgemeinen Formel

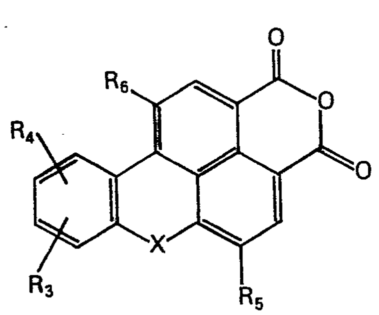

II

oder die Derivate der allgemeinen Formel

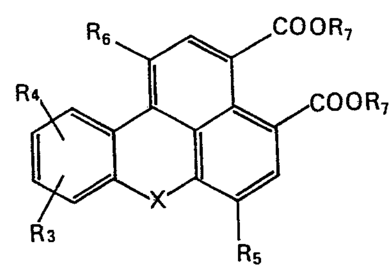

III

worin X, $R_3$, $R_4$, $R_5$ und $R_6$ die vorstehend genannten Bedeutungen haben und $R_7$ ein Wasserstoffatom oder eine Alkylgruppe bedeutet, mit einer Verbindung der Formel

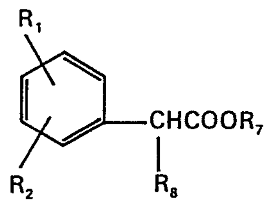

IV

und/oder

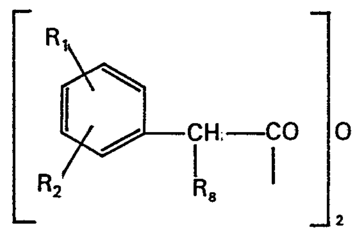

V

worin $R_1$, $R_2$ und $R_7$ die vorstehend genannten Bedeutungen haben und $R_8$ ein Wasserstoffatom oder eine Hydroxylgruppe bedeuten, in Gegenwart von Alkali auf Temperaturen zwischen etwa 150°C und etwa 260°C erhitzt und die so erhaltenen Verbindungen, bestehend aus dem Isomerengemisch der allgemeinen Formeln

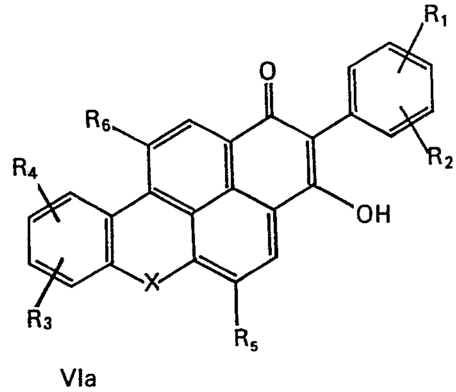

VIa

und

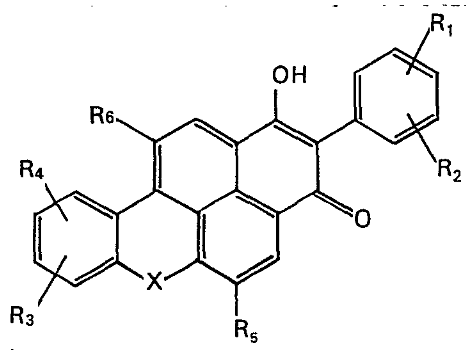

VIb

in denen X, $R_1$, $R_2$, $R_3$, $R_4$, $R_5$ und $R_6$ die vorstehend angegebenen Bedeutungen haben, alkyliert oder acyliert.

Die Kondensation der Verbindungen II bzw. III mit den Verbindungen der Formel IV bzw. V erfolgt in der Mehrzahl der Beispiele in der Schmelze, kann jedoch auch in einem polaren Lösemittel erfolgen, wie N-Methylpyrrolidon. Das so erhaltene Zwischenprodukt, das Isomerengemisch der Formeln VIa und VIb, wird in jedem Falle isoliert, bevor es der anschliessenden Alkylierung- oder Acylierungsreaktion zugeführt wird. Diese Zwischenprodukte zeigen jedoch ein ausgesprochen schlechtes Filtrationsverhalten, so dass dieser Isolierungsschritt einen erheblichen Aufwand an Zeit und Apparaten erfordert.

Es wurde nun gefunden, dass man die Verbindungen der Formel Ia und Ib in besonders vorteilhafter Weise herstellen kann, wenn man die Kondensations- und die Alkylierungs- bzw. Acylierungsreaktion ohne Isolierung der Zwischenprodukte durchführt. Es ist überraschend, dass diese erhebliche Verfahrensvereinfachung nicht mit einer verschlechterten Ausbeute oder Reinheit des Endprodukts erkauft werden muss.

Gegenstand der Erfindung ist somit ein Verfahren zur Herstellung der vorstehend charakterisierten Isomerengemische von Verbindungen der allgemeinen Formel Ia und Ib durch Erhitzen von Verbindungen der Formel II oder III mit Verbindungen der Formel IV und/oder V in Gegenwart von Alkali auf Temperaturen zwischen 150 und 260°C und anschliessendes Alkylieren oder Acylieren des Isomerengemisches der Formeln VIa und VIb, dadurch gekennzeichnet, dass man die Reaktionen in einem polaren organischen Lösemittel ohne Isolierung des Isomerengemisches der Formeln VIa und VIb durchführt.

Als polare Lösemittel kommen die in der DE-A 20 25 291 genannten Lösemittel, vorzugsweise N-Methylpyrrolidon, in Betracht. Selbstverständlich muss das Lösemittel bei den Reaktionstemperaturen stabil, auch gegen das anwesende Alkali, und gegen die Reaktionsteilnehemer inert sein, also nicht alkylierend oder acylierend wirken und nicht alkylierbar oder acylierbar sein. Vorzugsweise liegt der Siedepunkt des Lösemittels über der Reaktionstemperatur, da dann die Abtrennung flüchtiger Nebenprodukte vereinfacht wird.

Das für die Kondensation erforderliche Alkali kann ein Alkalihydroxid-carbonat oder -acetat sein, bevorzugt sind Kaliumacetat und Kaliumcarbonat.

Besonders bevorzugt erfolgt die Kondensationsreaktion in Gegenwart von Kaliumacetat oder Kaliumcarbonat in N-Methylpyrrolidon bei einer Temperatur von 200 bis 210°C. Die erhaltene Reaktionslösung kann dann unmittelbar mit dem Alkylierungs- oder Acylierungsmittel umgesetzt werden.

Wird die Kondensation in Gegenwart von Alkaliacetat durchgeführt, dann empfiehlt es sich, die hierbei frei werdende Essigsäure abzutrennen, falls eine Alkylierungsreaktion mit Alkylierungsmitteln beabsichtigt ist, die mit dieser Essigsäure reagieren können, beispielsweise Dialkylsulfaten. Andernfalls ist nicht nur ein erhöhter Einsatz an Alkylierungsmittel erforderlich, sondern der entstehende Essigester kann auch als Fällungsmittel für das Zwischenprodukt der Formeln VIa und VIb dienen.

Die Alkylierung und Acylierung erfolgt in der für phenolische bekannten Weise. Geeignete Alkylierungsmittel sind insbesondere die Dialkylsulfate, vor allem niedere Dialkylsulfate wie Dimethylsulfate oder Diäthylsulfat, Alkylhalogenide wie Methylchlorid oder Benzylchlorid, Alkylenoxide wie Äthylenoxid oderPropylenoxidund Arylsulfonsäureester wie p-Toluolsulfonsäuremethylester. Als Acylierungsmittel kommen aliphatische oder aromatische Carbon- oder Sulfonsäurechloride oder Carbonsäureanhydride, wie Acetylchlorid, Propionylchlorid, Benzoylchlorid, Benzolsulfonsäurechlorid oder Essigsäureanhydrid in Betracht.

Zur Isolierung des Endproduktes aus der Reaktionslösung wird diese zweckmässig abgekühlt und ein Fällungsmittel zugegeben. Als soche Fällungsmittel eignen sich mit dem Reaktionsmedium mischbare organische Flüssigkeiten, in denen der Farbstoff schwer löslich ist und die sich leicht vom Reaktionsmedium abtrennen lassen. Geeignet sind niedere Alkonale, insbesondere Methanol.

Die Farbstoffe werden in hoher Ausbeute und der für Fluoreszenzfarbstoffe erforderlichen hohen Reinheit erhalten.

In den folgenden Beispielen wird die Erfindung näher erläutert. Prozentangaben beziehen sich auf das Gewicht.

Beispiel 1

In einem Rührkessel wird eine Mischung aus 400 kg N-Methylpyrrolidon, 104,6 kg Phenylessigsäure und 62,8 kg Kaliumacetat auf 80°C erwärmt. Anschliessend werden 196 kg Benzothioxanthen-3,4-dicarbonsäureanhydrid zugegeben und die Mischung im Lauf von 2 Stunden auf 180°C erwärmt. Nach einer Nachrührzeit von 1 Stunde bei 180°C wird die Reaktionstemperatur auf 200–210°C gesteigert und 8–10 Stunden bei dieser Temperatur gerührt.

Nach Beendigung der Reaktion lässt man innerhalb von 30 Minuten bei 200°C 63 kg 50%ige

Schwefelsäure zulaufen. Das Reaktionsgemisch wird auf 120°C abgekühlt, bei etwa 120–130°C und etwa 65 bis 95 mbar (50–70 Torr) Essigsäure und Wasser bis zu einer Kopftemperatur von 130°C bei 80 mbar (60 Torr) abdestilliert.

Ist die Destillation beendet, werden 300 kg N-Methylpyrrolidon zugegeben und 68,2 kg Kaliumcarbonat eingetragen. Das Reaktionsgemisch wird 30 Minuten bei 120°C nachgerührt und dann auf 80 bis 85°C abgekühlt. Es werden weitere 14 kg Kaliumcarbonat zugegeben und bei 80 bis 85°C nachgerührt das Produkt anschliessend mit 400 kg Methanol bei 30–40°C gefällt und bei 15°C auf einer Drucknutsche isoliert. Man erhält in guter Ausbeute einen blaustichig-roten, stark fluoreszierenden Farbstoff, bestehend aus einem Isomerengemisch der Formeln

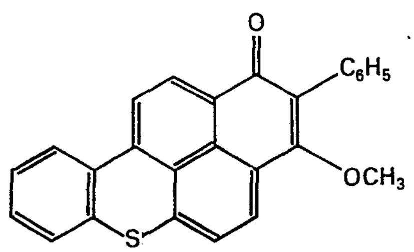

und

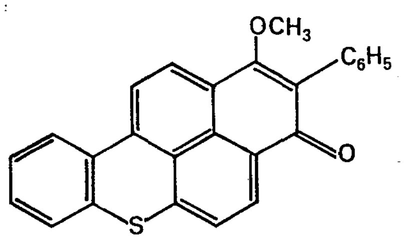

Beispiel 2

In einen Rührkessel werden 76 kg Benzothioxanthen-3,4-dicarbonsäureanhydrid, 37,5 kg Phenylessigsäure, 25 kg Kaliumacetat und 200 kg N-Methylpyrrolidon auf 180°C erwärmt. Nach einer Nachrührzeit von 1 Stunde bei 180°C wird auf 200°C erhitzt und 6 Stunden bei dieser Temperatur gerührt.

Ist die Reaktion beendet, wird auf 110°C gekühlt, innerhalb von 20 Minuten 75 kg Essigsäureanhydrid zugetropft und 2 Stunden bei 110°C nachgerührt. Anschliessend wird auf 20°C abgekühlt, 200 l Methanol zugegeben, 1 Stunde nachgerührt und das Produkt auf einer Drucknutsche isoliert. Es wird in guter Ausbeute ein reiner, blaustichig roter fluoreszierender Farbstoff erhalten, bestehend aus einem Isomerengemisch der Formeln

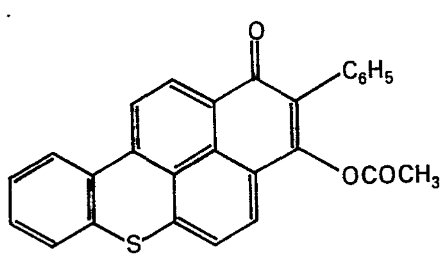

und

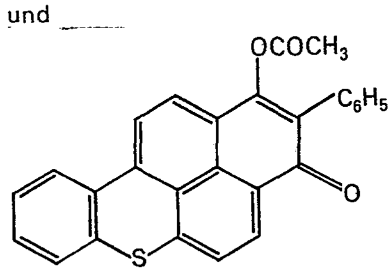

Beispiel 3

In einem Rührkessel werden 79,5 kg 10-Methoxy-benzoxanthen 3,4-dicarbonsäureanhydrid, 37,5 Phenylessigsäure, 25 kg Kaliumacetat und 200 kg N-Methylpyrrolidon vorgelegt. Das Reaktionsgemisch wird innerhalb von 2 Stunden auf 180°C erwärmt, 1 Stunde nachgerührt, dann schnell auf 200°C erhitzt und 6 Stunden bei dieser Temperatur nachgerührt.

Ist die Reaktion beendet, wird auf 110°C gekühlt, innerhalb von 20 Minuten 75 kg Essigsäureanhydrid zugetropft und 2 Stunden bei 110°C nachgerührt. Anschliessend wird auf 20°C abgekühlt, 200 l Methanol zugegeben, 1 Stunde nachgerührt und das Produkt auf einer Drucknutsche isoliert. Es wird in guter Ausbeute ein reiner, orangegelber, stark fluoreszierender Farbstoff erhalten, bestehend aus einem Isomerengemisch der Formeln

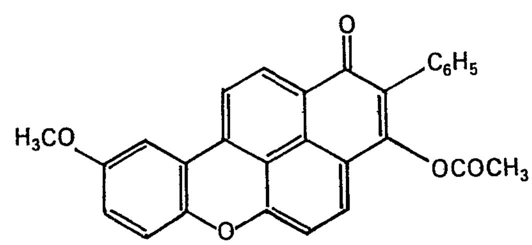

und

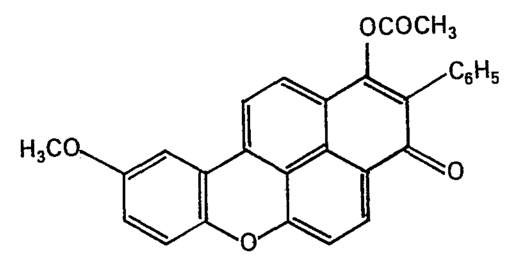

Beispiel 4

In einem Rührkessel wird eine Mischung aus 79,5 kg 10-Methoxybenzoxanthen-3,4-dicarbonsäureanhydrid, 37,5 kg Phenylessigsäure, 25 kg Kaliumacetat und 200 kg N-Methylpyrrolidon auf 180°C erwärmt. Nach einer Nachrührzeit von 1 Stunde bei 180°C wird weiter auf 200°C erhitzt und 8 Stunden bei dieser Temperatur nachgerührt.

Ist die Reaktion beendet, werden bei 190–200°C innerhalb von 25 Minuten 25,4 kg 50%ige Schwefelsäure zugegeben. Das Reaktionsgemisch wird auf 120°C abgekühlt, bei 120–160°C und etwa 65

bis 95 mbar (50–70 Torr) Essigsäure und Wasser bis zu einer Kopftemperatur von 160°C bei 80 mbar (60 Torr) abdestilliert.

Ist die Destillation beendet, werden 121 kg N-Methylpyrrolidon und bei 120 bis 123°C innerhalb von 25 Minuten 27,5 kg Kaliumcarbonat zugegeben. Das Reaktionsgemisch wird 30 Minuten bei 120°C nachgerührt und dann auf 80–85°C abgekühlt. Es werden weitere 5,6 kg Kaliumcarbonat zugegeben und bei 80°C mit 60,5 kg Dimethylsulfat innerhalb von 1–2 Stunden methyliert. Ist die Reaktion beendet, wird 2 Stunden bei 80–85°C nachgerührt, anschliessend das Produkt bei 65°C mit 161 kg Methanol gefällt, die Mischung auf 5–10°C abgekühlt, 1 Stunde nachgerührt und der Farbstoff auf einer Drucknutsche isoliert. Man erhält in guter Ausbeute einen orangegelben, fluoreszierenden Farbstoff, bestehend aus einem Isomerengemisch der Formeln

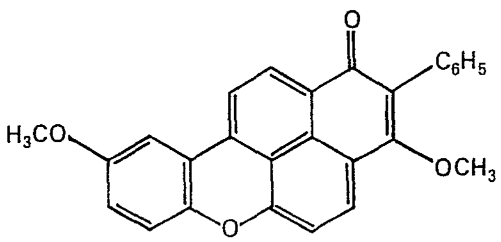

und

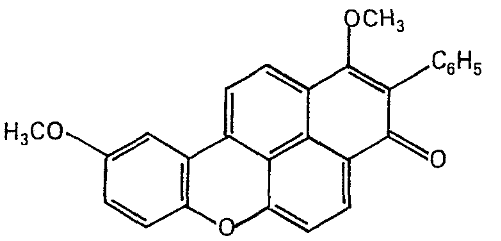

Beispiel 5

In einem Rührkessel wird eine Mischung aus 400 kg α-Chlornaphthalin, 104,6 kg Phenylessigsäure und 62,8 kg Kaliumacetat auf 80°C erwärmt. Anschliessend werden 196 kg Benzothioxanthensäure-3,4-dicarbonsäureanhydrid zugegeben und die Mischung im Lauf von 2 Stunden auf 180°C erwärmt. Nach einer Nachrührzeit von 1 Stunde bei 180°C wird das Reaktionsgemisch auf 200–210°C erhitzt und 8–10 Stunden bei dieser Temperatur gerührt.

Nach beendeter Reaktion werden, entsprechend Beispiel 1, innerhalb von 30 Minuten bei 190–200°C 63 kg 50%ige Schwefelsäure zugegeben. Das Reaktionsgemisch wird auf 120°C abgekühlt und bei 120–130°C und etwa 65 bis 95 mbar (50–70 Torr) werden Essigsäure und Wasser bis zu einer Kopftemperatur von 130°C bei 80 mbar (60 Torr) abdestilliert.

Ist die Destillation beendet, werden 300 kg α-Chlornaphthalin zugegeben und bei 80–85°C mit 160 kg Dimethylsulfat innerhalb von 1–2 Stunden methyliert. Ist die Reaktion beendet, wird 2 Stunden bei 80 bis 85°C nachgerührt und anschliessend das Produkt mit 400 kg Methanol bei 30°C–40°C gefällt und bei 15°C auf einer Drucknutsche isoliert. Man erhält in guter Ausbeute den blaustichig-roten, reinen, stark fluoreszierenden Farbstoff gemäss Beispiel 1.

Beispiel 6

In einem Rührkessel werden 76 kg Benzothioxanthen-3,4-dicarbonsäureanhydrid, 37,5 kg Phenylessigsäure, 25 kg Kaliumacetat und 200 kg Hexamethylphosphorsäuretrisamid zusammen auf 180°C erwärmt. Nach einer Nachrührzeit von 1 Stunde bei 180°C wird weiter auf 200°C erhitzt und 6 Stunden bei dieser Temperatur gerührt.

Ist die Reaktion beendet, wird auf 110°C gekühlt. Man lässt dann innerhalb von 20 Minuten 75 kg Essigsäureanhydrid zulaufen und rührt 2 Stunden bei 110°C nach. Anschliessend wird auf 20°C abgekühlt, 20 l Methanol zugegeben, 1 Stunde nachgerührt und auf einer Drucknutsche isoliert. Es wird in guter Ausbeute der reine, blaustichig-rote, fluoreszierender Farbstoff gemäss Beispiel 2 erhalten.

**Patentansprüche:**

1. Verfahren zur Herstellung von Isomerengemischen von Verbindungen der allgemeinen Formel Ia und Ib

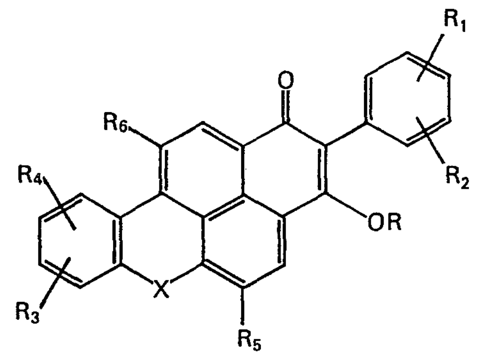

Ia

und

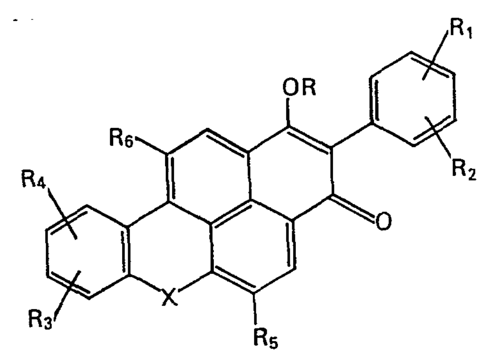

Ib

worin X ein Sauerstoff- oder Schwefelatom, R einen gegebenenfalls substituierten Alkylrest, Acylrest oder Benzolsulfonylrest, $R_1$ und $R_2$ Wasserstoff- oder Halogenatome, Alkyl-, Phenyl-, Acyl-, Acyloxy-, Carbalkoxy-, Alkoxy-, Nitro-, Alkylthio- oder Alkylsulfonylgruppen oder zusammen einen annellierten Benzolring, $R_3$ und $R_4$

Wasserstoff- oder Halogenatome, Phenylgruppen, Alkyl-, Alkoxy-, Carbalkoxygruppen mit 1 bis 4 C-Atomen oder Nitrilgruppen, $R_5$ und $R_6$ Wasserstoffatome oder gegebenenfalls substituierte Alkoxygruppen mit 1 bis 4 C-Atomen bedeuten, durch Erhitzen von Verbindungen der Formel II oder III

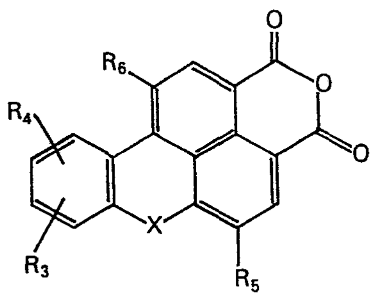

II

oder

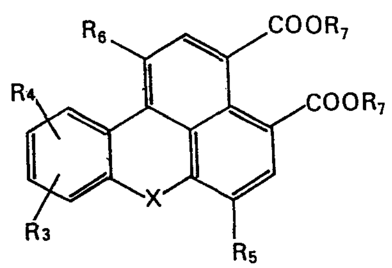

III

worin X, $R_3$, $R_4$, $R_5$ und $R_6$ die vorstehend genannten Bedeutungen haben und $R_7$ ein Wasserstoffatom oder eine Alkylgruppe bedeutet, mit Verbindungen der Formel IV und/oder V

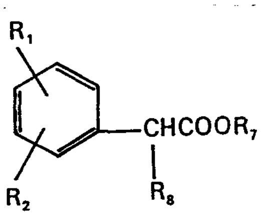

IV

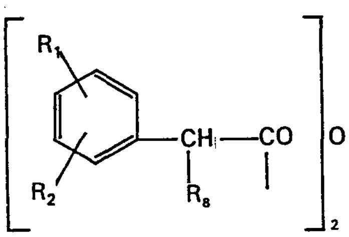

V

worin $R_1$, $R_2$ und $R_7$ die vorstehend genannten Bedeutungen haben und $R_8$ ein Wasserstoffatom oder eine Hydroxylgruppe bedeuten, in Gegenwart von Alkali auf Temperaturen zwischen 150° und 260°C und anschliessendes Alkylieren oder Acylieren des Isomerengemisches der Formeln VIa und VIb

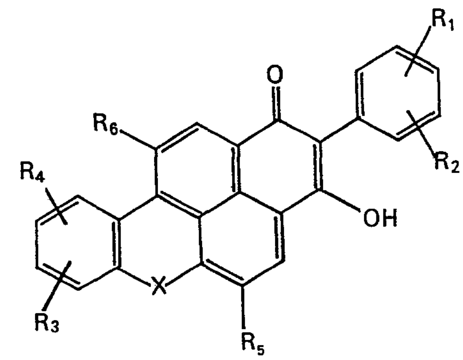

VIa

und

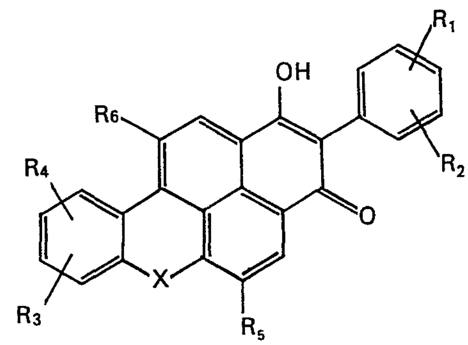

VIb

worin X, $R_1$, $R_2$, $R_3$, $R_4$, $R_5$ und $R_6$ die vorstehend genannten Bedeutungen haben, dadurch gekennzeichnet, dass man die Reaktion in einem polaren organischen Lösemittel ohne Isolierung des Isomerengemisches der Formeln VIa und VIb durchführt.

2. Verfahren nach Anspruch 1, dadurch gekennzeichnet, dass man als polares Lösemittel N-Methylpyrrolidon einsetzt.

3. Verfahren nach Anspruch 1 und 2, dadurch gekennzeichnet, dass die Kondensation bei 200–210°C durchgeführt wird.

4. Verfahren nach Anspruch 1 bis 3, dadurch gekennzeichnet, dass das Endprodukt aus der Reaktionslösung mit einem niederen Alkanol ausgefällt wird.

**Claims:**

1. Process for the manufacture of a mixture of isomers of the general formulae Ia and Ib

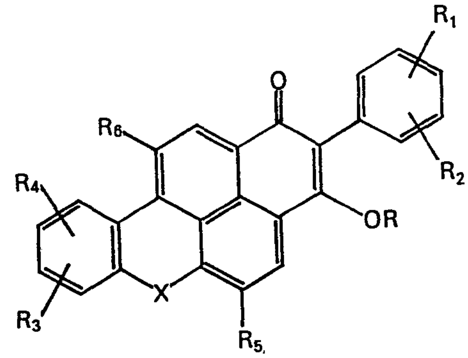

Ia

and

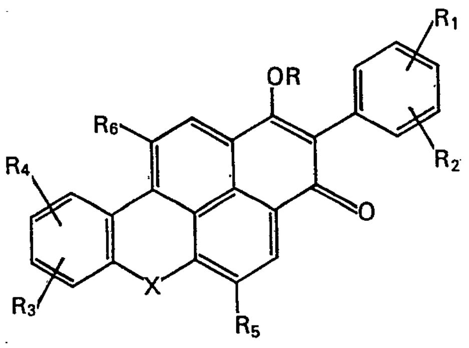

Ib

wherein X denotes an oxygen, or sulfur atom, R denotes an optionally substituted alkyl residue, acyl residue or benzenesulfonyl residue, $R_1$ and $R_2$ denote hydrogen or halogen atoms, alkyl, phenyl, acyl, acyloxy, carbalkoxy, alkoxy, nitro, alkylthio or alkylsulfonyl groups or they denote together an annellated benzene ring, $R_3$ and $R_4$ denote hydrogen or halogen atoms, phenyl groups alky, alkoxy, carbalkoxy groups with 1 to 4 C-atoms or nitrile groups, $R_5$ and $R_6$ denote hydrogen atoms or optionally substitued alkoxy groups with 1 to 4 C-atoms by heating to a temperature between 150 and 260°C in the presence of alkaline agents compounds the formula II or III

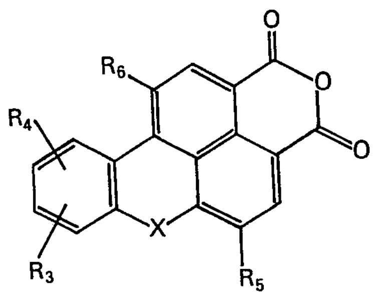

II

or

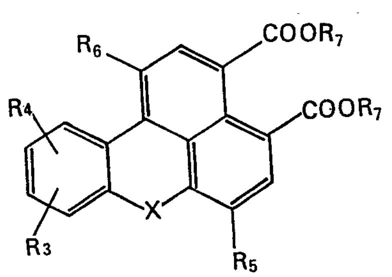

III

wherein X, $R_3$, $R_4$, $R_5$ and $R_6$ have the abovementioned meanings and $R_7$ is a hydrogen atom or an alkyl group, with a compound of the formulae IV and/ou V

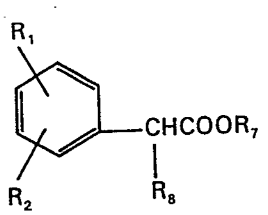

IV

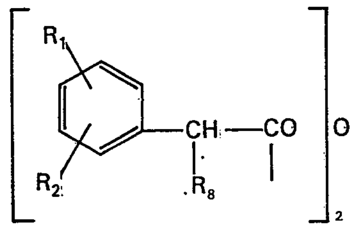

V

wherein $R_1$, $R_2$ and $R_7$ have the meanings given above, and $R_8$ is a hydrogen atoms or a hydroxy group, and by subsequent alkylating or acylating the mixture of isomers or the formulae VIa and VIb

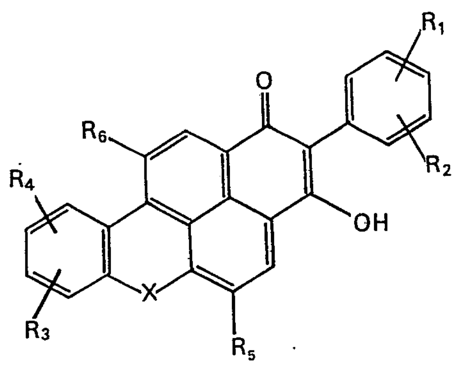

VIa

and

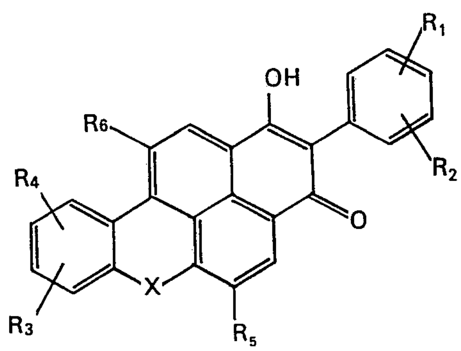

VIb

wherein X, $R_1$, $R_2$, $R_3$, $R_4$, $R_5$ and $R_6$ have the abovementioned meanings, characterized by performing the reaction in a polar organic solvent without isolation of the isomer mixture of the formulae VIa and VIb.

2. Process according to claim 1, characterized in that the polar organic solvent is N-methylpyrrolidone.

3. Process according to claims 1 and 2, characterized in that the condensation is performed at a temperature of 200 to 210°C.

4. Process according to claims 1 to 3, characterized in that the final is precipitated from the reaction solution with a lower alkanol.

## Revendications

1. Procédé de préparation de mélanges d'isomères composés répondant aux formules générales Ia et Ib

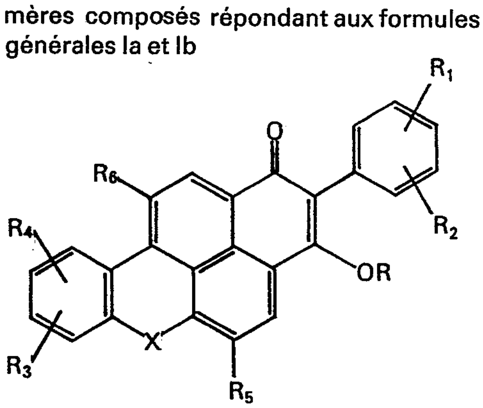

Ia

et

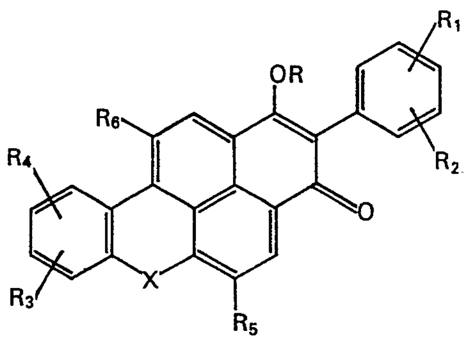

Ib

formules dans lesquelles X représente un atome doxygène ou de soufre, R un radical alkyle éventuellement substitué, un radical acyle ou un radical benzène-sulfonyle, $R_1$ et $R_2$ représentent chacun un atome d'hydrogène ou d'halogène ou un radical alkyle, phényle, acyle, acyloxy, alcoxycarbonyle, alcoxy, nitro, alkylthio ou alkylsulfonyle ou forment ensemble un noyau benzylique condensé, $R_3$ et $R_4$ représentent chacun un atome d'hydrogène ou d'halogène, un radical phényle, alkyle ou alcoxy, un radical alcoxycarbonyle contenant de 1 à 4 atomes de carbone ou un groupe cyano, et $R_5$ et $R_6$ représentent chacun un atome d'hydrogène ou un radical alcoxy contenant de 1 à 4 atomes de carbone et éventuellement substitué, par chauffage de composés répondant à l'une des formules II et III

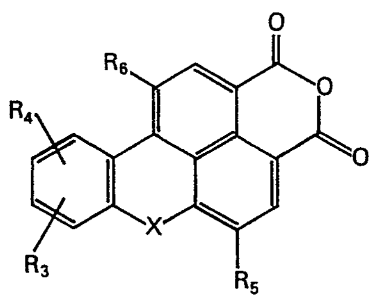

II

et

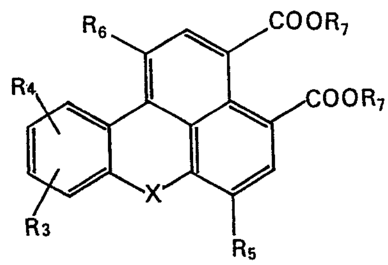

III

dans lesquelles X, $R_3$, $R_4$, $R_5$, et $R_6$ ont les significations précédemment données et $R_7$ représente un atome d'hydrogène ou un radical alkyle, avec des compesés de formules IV et/ou V

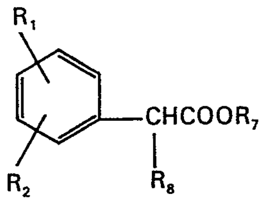

(IV)

et

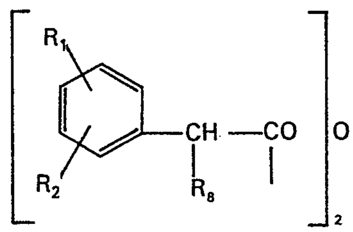

(V)

dans les quelles $R_1$, $R_2$ et $R_7$ ont les significations précédemment donées et $R_8$ représente un atome d'hydrogène ou un groupe hydroxy, en présence d'un alcali, à des températures comprises entre 150 et 260°C, puis alkylation ou acylation du mélange des isomères de formules VIa et VIb

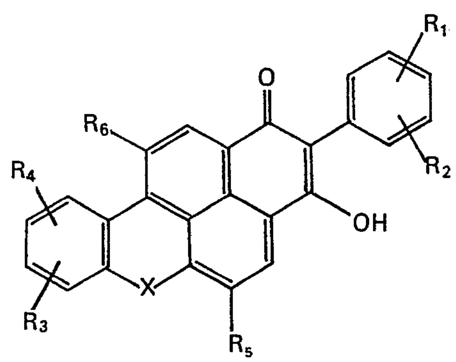

VIa

et

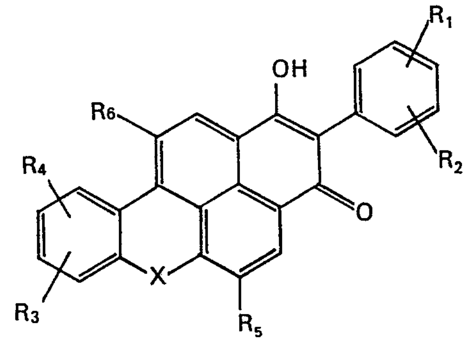

VIb

dans lesquelles X, $R_1$, $R_2$, $R_3$, $R_4$, $R_5$ et $R_6$ ont les significations précédemment donées, procédé caractérisé en ce qu'on efectue la réaction dans un solvant organique polaire sans isoler le mélange des isomères di formules VIa et VIb.

2. Procéde selon la revendication I, caractérisé en ce qu'on utilise la N-méthyl-pyrrolidone comme solvant polaire.

3. Procédé selon l'une des revendications 1 et 2, caractérisé en ce qu'on effectue la condensation à une température de 200-210°C.

4. Procédé selon l'une quelconque des revendications 1 à 3, caractérisé en ce qu'on fait précipiter le produit final, dans la solution réactionelle, au moyen d'un alcanol inférieur.